# EUROPÄISCHE PATENTSCHRIFT

(11) **EP 1 176 941 B1**
(45) Veröffentlichungstag und Bekanntmachung des Hinweises auf die Patenterteilung: **07.12.2005**
(21) Anmeldenummer: 00931086.3
(22) Anmeldetag: 28.04.2000
(51) Int. Cl.: A61K 7/32

(54) **MEHRPHASEN-ANTITRANSPIRANT-STIFT**
MULTI-PHASE ANTIPERSPIRANT STICK
BATON D'ANTITRANSPIRANT MULTIPHASE

(30) Priorität: 07.05.1999 DE 19921183
(43) Veröffentlichungstag der Anmeldung: 06.02.2002
(73) Patentinhaber: Henkel Kommanditgesellschaft auf Aktien, 40589 Düsseldorf (DE)
(72) Erfinder: BANOWSKI, Bernhard, D-40597 Düsseldorf (DE); KEMMESIES, Andrea, D-65185 Wiesbaden (DE); PÖPPL, Marion, D-41564 Kaarst (DE)
(86) Internationale Anmeldenummer: PCT/EP2000/003862
(87) Internationale Veröffentlichungsnummer: WO 2000/067712

(56) Entgegenhaltungen:
- EP-A- 0 000 604
- FR-A- 2 371 918

## Beschreibung

Die Erfindung betrifft eine kosmetische Zubereitung zur Bekämpfung von Achselnässe und unangenehmer Körpergerüche in Form eines Stiftes zur Abgabe einer bei Umgebungstemperatur formstabilen, bei Körpertemperatur auf der Haut verstreichbaren Masse, die zwei oder mehrere, unterschiedlich zusammengesetzte, verfestigte Phasen auf der Basis von flüchtigen Silikonölen und Geliermitteln enthalten.

Zweiphasen-Stiftzubereitungen auf Basis alkoholischer Seifengele sind seit langem z.B. aus DE-AS 1 122 221 bekannt. Solche Zubereitungen bieten die Möglichkeit, einzelne Komponenten, die mit anderen Komponenten unverträglich sind, in eine Phase des Stiftes einzubringen und auf diese Weise unerwünschte Wechselwirkungen mit den Komponenten der zweiten Phase zu verhindern. Auch kann man die Lagerbeständigkeit der Stiftpräparate dadurch erhöhen, daß man leichter flüchtige oder oxidationsempfindliche Komponenten in den Kern des Stiftes einbringt. Schließlich kann man in die Kern- und Hüllphase unterschiedliche Wirkstoffe einbringen oder die Wirkstoffe in unterschiedlicher Konzentration einbringen, um das anwendungstechnische Verhalten des Stiftes zu verbessern. So kann man z.B. für den Kern des Stiftpräparates eine Masse wählen, die wegen einer klebrigen oder weichen Konsistenz nicht als Hüllmasse geeignet ist.

Weitere Antitranspirant-Stiftzubereichungen mit benigstens zwei unterschiedlich Zusammengesetzten Phasen sind aus EP 0 000 604 und FR 2371918 bekannt.

Im vorliegenden Fall hat sich gezeigt, daß man den Kern auch als "Leistungskern" mit einer höheren Konzentration an schweißhemmendem Wirkstoff ausführen kann und auf diese Weise ein verbessertes Abriebverhalten bei der Anwendung erhält.

Gegenstand der Erfindung ist daher ein Stiftpräparat aus einer bei 40°C formstabilen, auf der Haut verstreichbaren und bei Temperaturen oberhalb von 40°C schmelzenden Masse, die aus zwei oder mehreren getrennten, unterschiedlich zusammengesetzten und konzentrisch als Kern und Hülle angeordneten verfestigten Phasen besteht, die ein flüssiges Öl und ein Gelier- oder Festigungsmittel enthalten, wie in den Ansprüchen definiert.

Das erfindungsgemäße Stiftpräparat kann man darüber hinaus durch unterschiedliche Transparenz, Färbung oder Pigmentierung der Phasen ästhetisch sehr attraktiv gestalten.

Verfestigte Phasen im Sinne der vorliegenden Erfindung sind Zusammensetzungen, die ein flüssiges Öl, ausgewählt aus niedrig-siedenden und flüchtigen cyclischen Silikonen und niedrig-siedenden, linearen Polydimethylsiloxanen mit 3 - 10 Si(CH₃)₂O-Einheiten, und ein Gelier- oder Festigungsmittel enthalten. Von besonderem Vorteil für die Anwendung in Deodorant-Stiften sind die niedrig-siedenden und flüchtigen cyclischen Silikone, da sie auf der Haut keine schmierigen Rückstände hinterlassen. Geeignet sind aber auch die niedrig-siedenden, linearen Polydimethylsiloxane mit 3 - 10 Si(CH₃)₂O-Einheiten. Ein bevorzugt cyclisches Silikon ist z.B. das Cyclopentadimethylsiloxan.

Die erfindungsgemäßen Stiftpräparate enthalten in der verfestigten Phase 20 - 70 Gew.-% flüssige Silikonöle und 10 - 30 Gew.-% eines Gelier- oder Festigungsmittels aus der Gruppe der gesättigten Fettalkohole, der gesättigten Triglyceride, der Wachsester oder eines Gemisches davon.

Als Geliermittel können darüber hinaus auch andere Stoffe verwendet werden, z.B. Dibenzylidensorbit, N-acylierte Fettsäureamide und N-acylierte Aminosäureamide. Auch bestimmte öllösliche Polymerisate, z.B. Polyacrylamide, Fettsäure-Seifen (z.B. Magnesiumstearat oder Aluminiumstearat) oder 12-Hydroxystearinsäure und deren Ester oder Salze sind als Gelbildner geeignet. Bevorzugt geeignete Fettalkohole sind die linearen, gesättigten Fettalkohole mit 14 - 40 C-Atomen, insbesondere Cetyl- und Stearylalkohol. Geeignete Fettsäureglyceride sind z.B. gehärtetes Palmfett, gehärteter Rindertalg, gehärtetes Rizinusöl, Trilaurin oder Tristearin. Geeignete Wachsester sind z.B. Cetylpalmitat, Stearylstearat oder Laurylbehenat.

Neben den genannten Gelier- oder Festigungsmitteln kann die Gelphase auch geringere Mengen an flüssigen Ölkomponenten, z.B. flüssige Fettsäureester, Anlagerungsprodukte von 1 - 30 Mol Propylenoxid an Fettalkohole mit 4 - 18 C-Atomen, Paraffinöle oder verzweigte Fettalkohole, Dialkylether, z.B. Di-n-octylether oder Lauryl-methylether enthalten.

Schließlich kann die Gelphase zusätzlich zu den genannten Komponenten 1 - 15 Gew.-% eines nichtionischen Tensids enthalten, das die Abwaschbarkeit der Produkte von der Haut erleichtert. Bevorzugt sind dafür die bei 20°C festen Ethylenoxid-Addukte geeignet, z.B. an Fettalkohole, an Fettsäurepartialglyceride, an Sorbitanfettsäureester oder an andere Lipidmoleküle, die eine freie Hydroxyl- oder Carboxylgruppe tragen, die sich mit Ethylenoxid umsetzen läßt. Bevorzugt geeignete nichtionische Tenside sind z.B. das Anlagerungsprodukt von 10 bis 30 Mol Ethylenoxid an ein Cetyl-/Stearylalkohol-Gemisch, z.B. die Handelsprodukte Eumulgin® B1, B2 und B3 (Henkel KGaA). Weitere oberflächenaktive Stoffe, die sich erfindungsgemäß eignen, sind z.B. die Anlagerungsprodukte von 1 - 30 Mol Ethylenoxid an Methylglucosid-Fettsäureester sowie Alkyl-(oligo)-glucoside und Anlagerungsprodukte von 1 - 30 Mol Ethylenoxid an diese.

Darüber hinaus enthalten die Gelphasen Duftstoffe und gegebenenfalls kosmetische oder dermatologische Wirkstoffe.

Als Duftstoffe bzw. Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen, z.B. die Ionone ∝-Isomethylionen und Methyl-cedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Von besonderer Bedeutung für ein Deodorant/Antitranspirant ist der Gehalt an deodorierenden und/oder schweißhemmenden Wirkstoffen. Als deodorierende Wirkstoffe eignen sich vor allem antimikrobielle Wirkstoffe, die die schweißzersetzenden Keime hemmen und Enzyminhibitoren, welche die schweißzersetzenden Esterase- bzw. Lipase-Enzyme blockieren. Geeignete antimikrobielle Stoffe sind z.B. 2,4,4'-Trichlor-2'-hydroxydiphenylether (Triclosan®), Chlorhexidingluconat, Phenoxyethanol, 1,5-Pentandiol, 1,6-Hexandiol, antimikrobielle ätherische Öle oder Farnesol. Geeignete esterasehemmende Wirkstoffe sind z.B. Triethylcitrat oder Triacetin.

Als schweißhemmende Stoffe werden vor allem adstringierende bzw. proteinkoagulierende Salze wie z.B. die im wäßrigen Medium stark hydrolysierenden Aluminium-, Zink- und Zirkoniumsalze eingesetzt. Solche geeigneten antihydrotisch wirksamen Salze sind z.B. Aluminiumchlorid, Aluminiumchlorhydrat, Aluminiumdichlorhydrat, Aluminiumsesquichlorhydrat und deren Komplexverbindungen mit 1,2-Propylenglycol, Aluminiumhydroxyallantoinat, Aluminiumchlorid-tartrat, Natrium-Aluminiumchlorhydroxylactat, Aluminium-Zirkonium-trichlorhydrat, Aluminium-Zirkonium-tetrachlorhydrat, Aluminium-Zirkoniumpentachlorhydrat und deren Komplexverbindungen mit Aminosäuren, z.B. mit Glycin.

Ein wesentliches Merkmal der vorliegenden Erfindung besteht nun darin, daß die deodorierenden oder antihydrotischen Wirkstoffe in der Phase, die als Kern des Stiftes angeordnet ist, in einer Weise enthalten sind, die sich in Art und Menge von der Hüllphase unterscheidet. Bevorzugt enthält die Kernphase die 15 - 40 Gew.-% und die Hüllphase weniger als 18 Gew.-% eines schweißhemmenden, adstringierenden Salzes, wobei die Konzentration in der Kemphase wenigstens um 30 % höher, bezogen auf die Konzentration in der äußeren bzw. Hüllphase, ist. Die Hüllphase kann z.B. auch völlig frei von schweißhemmenden Salzen sein und eventuell einen antimikrobiellen deodorierenden Wirkstoff enthalten.
Auch kann die Hüllphase gegebenenfalls einen höheren Duftstoffgehalt aufweisen als die Kernphase.

Schließlich kann das erfindungsgemäße Stiftpräparat auch weitere Hilfsstoffe und Zusätze zur Verbesserung der Konsistenz, der Haltbarkeit, der anwendungstechnischen Eigenschaften und der Verträglichkeit enthalten. Solche Hilfsmittel sind z.B. Antioxidantien, Komplexbildner, inerte Füllstoffe, z.B. Schichtsilikate, Talkum, Kieselsäure, feinteilige Polymerpulver oder kosmetische Wirkstoffe, z.B. Vitamine, Allantoin, entzündungshemmende oder hautweichmachende Stoffe. Bevorzugt sind pulverförmige anorganische und organische Füllstoffe in einer Menge von 0,1 - 10 Gew.-% enthalten.

In einer bevorzugten Ausführung liegt das erfindungsgemäße Stiftpräparat in Form von zwei (oder mehr) konzentrisch angeordneten Gelphasen vor, deren innere bzw. Kemphase einen deodorierenden oder schweißhemmenden Wirkstoff enthält und sich in der Art und Menge dieses Wirkstoffs von der Hüllphase unterscheidet. Der Kern muß prinzipiell nicht zylindrisch geformt sein, sollte aber säulenförmig sein und kann einen beliebigen Querschnitt aufweisen. Aus Gründen der Fertigungstechnik kann es bevorzugt sein, daß die Phasen parallel zur Längsachse des Stiftes angeordnet sind.

In einer bevorzugten Ausführung enthält die Hüllphase einen Farbstoff oder ein Pigment, das sich in Art und Menge von den entsprechenden Komponenten in der Kemphase unterscheidet. Die äußere Phase (Hüllphase) ist bevorzugt gefärbt und stärker parfümiert als der Kern. Auf diese Weise werden ästhetisch besonders ansprechende Stiftpräparate erhalten. Bevorzugt eignen sich für diesen Zweck solche Farbstoffe, die in dem flüssigen Öl unlöslich sind und nicht über die Phasengrenze ausbluten.
Am besten eignen sich daher organische und anorganische Pigment-Farbstoffe, z.B. FD&C Red No 40, Al-Lake (C.I. 16035:1), D&C Red No 30 (C.I. 73360) D&C Red No 7 (C.I. 15850:1), C.I. Pigment Red 49:1 (C.I. 15630:2), C.I. Pigment Red 5 (C.I. 12490), D&C Yellow No 10 Al-Lake (C.I. 47005:1), C.I. Pigment Green 17 (C.I. 77288), C.I. Pigment Blue 29 (C.I. 77007) FD&C Blue No 1 Al-Lake (C.I. 42090:2), C.I. Pigment Blue 15 (C.I. 74160)

C.I. Pigment Blue 27 (C.I. 77510). Eisenoxid-gelb (C.I. 77492), Eisenoxid-rot (C.I. 77491), Eisenoxid-schwarz (C.I. 77499) und Titan-weiß (C.I. 77891).

Kern und Hülle können prinzipiell in beliebigen Mengenverhältnissen zueinander vorliegen, etwa von 1 : 5 bis 5 : 1. Bevorzugt stellt die Kernphase aber einen Anteil von 20 - 40 Gew.-% der gesamten Stiftmasse dar.

Die erfindungsgemäßen Stiftpräparate kann man z.B. in der Weise produzieren, daß man zunächst den Kern durch Gießen des in der Wärme flüssigen Gels in eine Form, Erkaltenlassen und Gelierung und Entformen herstellt und dann die Hülle durch Einbringen des Kerns in eine breitere Form, Eingießen der in der Wärme verflüssigten Gelphase für die Hülle in den Zwischenraum zwischen Kern und Formwand, Erkaltenlassen und Gelierung und Entformen herstellt.

Ein analoges Verfahren besteht darin, daß man zunächst die Hülle durch Eingießen des verflüssigten Gels in eine Ringform mit entfernbarem zylindrischen Kernstück, Erkaltenlassen unter Gelierung und Entfernen des Kernstücks herstellt und dann die verflüssigte Gelmasse des Kerns in den zylindrischen Hohlraum der so hergestellten Hülle eingießt und erstarren läßt.

In diesem Falle kann man nämlich prinzipiell die aus der Technologie der Seifenherstellung bekannten kontinuierlichen Verfahren zur Herstellung von mehrfarbigen Seifensträngen aus unterschiedlich gefärbten Seifenmassen einsetzen, um einen Strang aus zwei oder mehr verschiedenen Gelmassen herzustellen, der in Stifte beliebiger Länge geschnitten werden kann. Solche Verfahren zur Herstellung von Strängen aus konzentrisch angeordneten Phasen sind z.B. die aus AT-PS 198 501 oder DE-AS-2 526 917 bekannten Koaxial-Strangpreßverfahren. Andere Verfahren zur Herstellung mehrphasiger Stränge aus parallel zur Längsachse angeordneten (aber nicht konzentrischen) Phasen sind in US 3,268,970 beschrieben.

Die auf diese Weise hergestellten Mehrphasen-Stiftpräparate werden bevorzugt in eine Hülse mit einem entlang der Hülsenachse durch Schub-, Dreh- oder Druckmechanik verschiebbaren Bodenkolben eingebracht, wie sie für Deodorant-Stifte und andere kosmetische Stifte üblich sind. Dadurch wird eine bequeme Anwendung des Stifts ohne direkten Kontakt der Finger mit der Stiftmasse ermöglicht.

Die folgenden Beispiele sollen die Erfindung näher erläutern:

### Beispiele

Antitranspirant-Stiftmassen werden nach den in der Tabelle aufgeführten Rezepturen angefertigt.

| Zusammensetzung | K 1 | K 2 | H1 / K3 | H 2 |
|---|---|---|---|---|
| Cyclomethicone d 5 | 40,3 | 40,3 | 54,68 | 54,97 |
| Witconol® APM | -- | 11,0 | -- | 11,0 |
| Eumulgin® B1 | 5,0 | - | 5,0 | -- |
| Cutina® HR | 2,0 | 5,0 | 2,0 | 5,0 |
| Cetylalkohol | 5,0 | -- | 5,0 | -- |
| Stearylalkohol | 12,0 | 20,0 | 12,0 | 20,0 |
| Vitamin E-acetat | 0,4 | 0,4 | -- | - |
| Talkum Pharma G | 3,0 | 8,0 | 3,0 | 8,0 |
| Aerosil® R 972 | 2,0 | -- | 2,0 | -- |
| Al-Hydroxychlorid-Pulver | 30,0 | 15,0 | 15,0 | -- |
| Parfümöl | 0,3 | 0,3 | 1,3 | 1,0 |
| Farbstoff | -- | -- | 0,02 | 0,03 |

### 1. Herstellung der Gelphasen

Cyclomethicone d5, Witconol® APM, Eumulgin® B1, Cutina® HR, die Fettalkohole und Aerosil® R 972 wurden bei 70°C unter Rühren miteinander vermischt. Nach Abkühlung auf 65°C wurde das Al-Hydroxychlorid-Pulver, Talkum und Vitamin E-acetat sowie der Farbstoff zugegeben. Nach Abkühlung auf 60°C wurde das Parfumöl untergerührt.

### 2. Herstellung des Stiftpräparates

Eine entformte Stiftmasse gemäß K1 wurde als Kern in eine übliche Stifthülse zentriert eingesetzt. Dann wurde die Stiftmasse H1 hergestellt und bei 60°C in den Zwischenraum zwischen Kern und Hülsenwand eingegossen.

Nach Aufschrauben der Verschlußkappen wurden die Stifthülsen umgedreht, d.h. auf den Kopf gestellt, damit die noch nicht erstarrte Stiftmasse in der Verschlußkappe eine glatte Oberfläche bildet. Nach Abkühlung auf 20°C wurde ein attraktiv aussehender 2-Phasen-Stift mit blau gefärbter äußerer Phase und weißem Kern erhalten.

In gleicher Weise wurden 2-Phasen-Stifte aus den Stiftmassen
K1 (Kern) + H2 (Hülle)
K1 (Kern) + H1 (Hülle)
K2 (Kern) + H2 (Hülle)
K3 (Kern) + H2 (Hülle)
hergestellt. Es wurden stets 2-Phasen-Stifte mit blau gefärbter Hüllphase und weißer bzw. heller eingefärbter Kernphase erhalten, die bei Lagerung bei 40°C über 1 Woche keine Veränderung zeigten.

Es wurden die folgenden Handelsprodukte eingesetzt:

| | |
|---|---|
| Dow Corning 345 Fluid: | Cyclomethicone d5 (Decamethylcyclopentasiloxan) |
| Witconol®APM: | PPG3-Myristylether |
| Cutina®HR: | Hydr. Rizinusöl |
| Eumulgin B1: | Ceteareth-12 (Cetylstearylalkohol +12 EO) |
| Aerosil® R972: | pyrogene Kieselsäure, hydrophob |

## Patentansprüche

1. Stiftpräparate aus einer bei 40°C formstabilen, auf der Haut verstreichbaren und bei Temperaturen oberhalb 40°C schmelzenden Masse, die aus wenigstens zwei getrennten, unterschiedlich zusammengesetzten und konzentrisch als Kern und Hülle angeordneten verfestigten Phasen besteht, die ein flüssiges Öl und ein Gelier- oder Festigungsmittel enthalten, wobei die Kernphase, die einen deodorierenden und/oder schweißhemmenden Wirkstoff enthält, sich in der Art und/oder Menge dieser Wirkstoffe von der Hüllphase unterscheidet, **dadurch gekennzeichnet, dass** die verfestigten Phasen 20 - 70 Gew.-% eines flüssigen Silikonöls, ausgewählt aus niedrig-siedenden und flüchtigen cyclischen Silikonen und niedrig-siedenden linearen Polydimethylsiloxanen mit 3 - 10 Si(CH₃)₂O-Einheiten, und 10 -30 Gew.-% eines Gelier- oder Festigungsmittels aus der Gruppe der gesättigten Fettalkohole, der gesättigten Triglyceride, der Wachsester oder eines Gemisches davon sowie pulverförmige anorganische Füllstoffe in einer Menge von 0,1 - 10 Gew.-% enthalten.

2. Stiftpräparate gemäß Anspruch 1, **dadurch gekennzeichnet, dass** die Kemphase 15 - 40 Gew.-% und die Hüllphase weniger als 18 Gew.-% eines schweißhemmenden, adstringierenden Salzes enthält, wobei die Konzentration in der Kemphase wenigstens um 30 % höher, bezogen auf die Konzentration in der Hüllphase, ist.

3. Stiftpräparate gemäß einem der Ansprüche 1 oder 2, **dadurch gekennzeichnet, dass** die verfestigten Phasen zusätzlich 1 - 15 Gew.-% eines nichtionischen Tensids, bevorzugt eines bei 20°C festen Ethylenoxidaddukts, enthalten.

4. Stiftpräparate gemäß einem der Ansprüche 1 - 3, **dadurch gekennzeichnet, dass** die Hüllphase einen Farbstoff oder ein Pigment enthält, das sich in Art und/oder Menge von der Kemphase unterscheidet.

5. Stiftpräparate gemäß einem der Ansprüche 1 - 4, **dadurch gekennzeichnet, dass** die Kemphase einen Anteil von 20 - 40 Gew.% der gesamten Stiftmasse darstellt und die Hüllphase einen im flüssigen Öl unlöslichen Pigmentfarbstoff enthält.

## Claims

1. Stick preparations of a mass which is dimensionally stable at 40°C, can be spread on the skin and melts at temperatures above 40°C and which consists of at least two separate, solidified phases of different composition concentrically arranged as core and shell which contain a liquid oil and a gelling or solidifying agent, wherein the core phase which contains a deodorant and/or perspiration-inhibiting active differs from the shell phase in the type and/or quantity of these active principles, **characterized in that** the solidified phases contain 20 to 70 % by weight of a liquid silicone oil , selected from low-boiling and volatile cyclic silicones and low-boiling linear polydimethyl siloxanes containing 3 to 10 Si(CH₃)₂O units, and 10 to 30 % by weight of a gelling or solidifying agent from the group of saturated fatty alcohols, saturated triglycerides, wax esters or a mixture thereof, and powder-form inorganic fillers in a quantity of 0.1 to 10 % by weight.

2. Stick preparations as claimed in claim 1, **characterized in that** the core phase contains 15 to 40 % by weight and the shell phase less than 18 % by weight of a perspiration-inhibiting astringent salt, the concentration in the core phase being at least 30 % higher, based on the concentration in the shell phase.

3. Stick preparations as claimed in any of claims 1 or 2, **characterized in that** the solidified phases additionally contain 1 to 15 % by weight of a non-ionic surfactant, preferably an ethylene oxide adduct solid at 20°C.

4. Stick preparations as claimed in any of claims 1 to 3, **characterized in that** the shell phase contains a dye or a pigment which differs in type and/or quantity from the core phase.

5. Stick preparations as claimed in any of claims 1 to 4, **characterized in that** the core phase makes up 20 to 40 % by weight of the stick as a whole and the shell phase contains a pigment insoluble in the liquid oil.

## Revendications

1. Préparations en bâtonnet constituées d'une masse de forme stable à 40°C, pouvant être étalées sur la peau et fondant à des températures supérieures à 40°C, qui sont constituées d'au moins deux phases solidifiées séparées, de composition différente et disposées concentriquement sous forme de noyau et d'enveloppe, contenant une huile liquide et un agent de gélification ou de solidification, dans lesquelles la phase noyau qui contient une matière active déodorante et/ou inhibant la transpiration se différencie de la phase enveloppe en nature et/ou en quantité de ces matières actives, **caractérisées en ce que** les phases solidifiées contiennent de 20 à 70% en poids d'une huile de silicone liquide choisie parmi les silicones cycliques à bas point d'ébullition et volatiles, et des polydiméthylsiloxanes linéaires à bas point d'ébullition comportant de 3 à 10 unités Si(CH₃)₂O, et de 10 à 30% en poids d'un agent de gélification ou de solidification du groupe des alcools gras saturés, des triglycérides saturés, des esters cireux ou d'un de leurs mélanges, ainsi que des charges inorganiques pulvérulentes en une quantité de 0,1 à 10% en poids.

2. Préparations en bâtonnet selon la revendication 1, **caractérisées en ce que** la phase noyau contient de 15 à 40% en poids et la phase enveloppe moins de 18% en poids d'un sel astringent inhibant la transpiration, la concentration dans la phase noyau étant d'au moins environ 30% supérieure, par rapport à la concentration dans la phase enveloppe.

3. Préparations en bâtonnet selon l'une des revendications 1 ou 2, **caractérisées en ce que** les phases solidifiées contiennent en outre de 1 à 15 % en poids d'un agent tensioactif non ionique, de préférence d'un produit d'addition d'oxyde d'éthylène solide à 20°C.

4. Préparations en bâtonnet selon l'une des revendications 1 à 3, **caractérisées en ce que** la phase enveloppe contient un colorant ou un pigment qui se différencie en nature et/ou en quantité de la phase noyau.

5. Préparations en bâtonnet selon les revendications 1 à 4, **caractérisées en ce que** la phase noyau contient une proportion de 20 à 40% en poids de l'ensemble de la masse du bâtonnet et **en ce que** la phase enveloppe contient un colorant de pigmentation insoluble dans l'huile liquide.
